# EUROPEAN PATENT SPECIFICATION

(11) **EP 3 922 370 B1**
(45) Date of publication and mention of the grant of the patent: **25.10.2023**
(21) Application number: 21178630.6
(22) Date of filing: 09.06.2021
(51) Int. Cl.: B09B 3/00, B09C 1/10, C05F 11/08, C12N 1/14

(54) **METHOD AND SOIL IMPROVER FOR BREAKING DOWN UNWANTED FIBERS IN THE SOIL, IN PARTICULAR ASBESTOS FIBRES**
VERFAHREN UND BODENVERBESSERUNGSMITTEL ZUM ABBAU UNERWÜNSCHTER FASERN IM BODEN, INSBESONDERE ASBESTFASERN
PROCÉDÉ ET PRODUIT D'AMENDEMENT DU SOL POUR LA DÉCOMPOSITION DE FIBRES INDÉSIRABLES DANS LE SOL, EN PARTICULIER DE FIBRES D'AMIANTE

(30) Priority: 09.06.2020 NL 2025792
(43) Date of publication of application: 15.12.2021
(73) Proprietor: Natural Soil Improvement BV, 6811 LJ Arnhem (NL)
(72) Inventor: BADE, Tom, 6815 HL Arnhem (NL)
(74) Representative: Verhees, Godefridus Josephus Maria

(56) References cited:
- WO-A1-2007/111479
- WO-A1-2013/090628
- US-A1- 2016 316 760
- BHATTACHARYA SHABORI ET AL: "Fungal weathering of asbestos in semi arid regions of India", ECOTOXICOLOGY AND ENVIRONMENTAL SAFETY, ACADEMIC PRESS, SAN DIEGO, CA, US, vol. 124, 2 November 2015 (2015-11-02), pages 186-192, XP029338666, ISSN: 0147-6513, DOI: 10.1016/J.ECOENV.2015.10.022

## Description

### Technical field of the invention

The invention relates to a method for breaking down asbestos fibers in the soil by introducing one or more of the following fungi into the soil to be cleaned: Verticillium leptobactrum, Talaromyces flavus, Trametes versicolor, Aspergillus tubingenesis and Postia stictica.

Asbestos is a collective name for a number of naturally occurring minerals that belong to the silicates. Raw asbestos consists of elongated bundles made up of crystals that exhibit longitudinal cleavage. The crystals are called asbestos fibers.

Asbestos is a natural product, consisting of rock and minerals with tough, non-combustible fibers. It is a mineral that is mined in South America, Russia and Canada, among others. There are different types of asbestos minerals. Asbestos fibers can be divided into two main groups:
- the spiral or serpentine ones, including chrysotile (or white asbestos)
- the straight or amphibolic ones, including crocidolite (blue asbestos), amosite (brown asbestos), anthophyllite (yellow), tremolite (grey) and actinolite (green).

It is well known that asbestos fibers play a role in the development of asbestos cancer or mesothelioma. The literature shows that in particular iron can be extracted from the fibers by biological organisms and that the released iron is suspected to be the cause of the formation of free radicals, which in turn cause oxidative stress. This oxidative stress could in turn be the cause of the development of mesothelioma.

### Background of the invention

A method according to the preamble of claim 1 is known fromBHATTACHARYA SHABORI ET AL: "Fungal weathering of asbestos in semi arid regions of India", ECOTOXICOLOGY AND ENVIRONMENTAL SAFETY, ACADEMIC PRESS, SAN DIEGO, CA, US, vol. 124, 2 November 2015 (2015-11-02), pages 186-192, XP029338666, ISSN: 0147-6513, DOI: 10.1016 / J.ECOENV.2015.10.022. The fungal species Aspergillus tubingenesis and Coemansia reversa are known to be able to extract iron from asbestos. These fungi appear to be able to remove iron from the dangerous blue asbestos in particular.

A lot of research is being done worldwide into the cost-effective demolition of asbestos, but it turns out to be extremely difficult to find a solution. Especially the combination of safety, effectiveness and not too high costs appears to be very difficult to achieve.

Microorganisms such as fungi need iron for their metabolism. When this iron does not come in sufficiently with the normal food intake, other mechanisms are used. Various micro-organisms have even developed specific functions for this.

It has been shown that iron in certain fungi increases significantly after cultivation in the presence of crocidolite asbestos. This increase is due to the presence of iron as a component of asbestos fibers. As a result, iron in those fibers decreases, resulting in a loss of integrity of the fibers.

The reduction in iron allows less iron to be released from the fibers that have been inhaled and, as a result, lower or no oxidative stress would occur. The direct consequence of this is that asbestos fibers that have been in long-term contact with certain micro-organisms (fungi) lose their carcinogenic (cancer-causing) properties.

From WO2007/111479A1 it is known to introduce a soil improver having the capability to restore the nutrient level of the soil and to remove heavy metals in the soil. From US2016/316760A1 it is known to use plants and fungi which are commonly hosted by the plant in order to improve agricultural traits of the plant.

### Summary of the invention

An object of the invention is to improve the known method. To this end, the method according to the invention is characterized in that fungi are also introduced into the soil which naturally belong to a plant that has a high biomass production and that this plant is planted in the soil, whereby no nutrients are added to the soil. Some fungi are known to break down asbestos. By exhausting the soil (removing nutrients), these fungi will extract the Fe and Mg and any Si present in the unwanted fibers from these fibers. This is done by the amino acids secreted by the fungi. Fe and Mg (and Si) act as necessary trace elements in the unwanted fiber. By removing the Fe and Mg (and Si) from the fibers, the fiber structure is broken down. By planting a plant with a high biomass production in the soil, nutrients are extracted from the soil. The fungi that break down the unwanted fibers must pass on the breakdown material to the fungi naturally belonging to this plant.

The plant with high biomass production is preferably harvested periodically. As a result, the nutrients are removed from the soil more quickly, so that the fungi break down the unwanted fibers more quickly.

As a plant with high biomass production, the Miscanthus plant is preferably planted in the soil and the fungi naturally associated with this plant are introduced into the soil. Miscanthus is a plant that has a very high biomass production and is therefore very suitable for quickly depleting the soil.

A further embodiment of the method according to the invention is characterized in that one or more of the following plants are furthermore arranged in the soil to be cleaned: Hemp, Sorghum, bamboo, hops and nettles. Due to the extremely high root density they possess, these plants will completely deplete the nutrients and minerals in the soil. The combination of acids, siderophores and other secondary metabolites by plant and microorganism, together with the continuous uptake of metals and minerals by the plant causes the "disappearance" of organic and mineral components from the soil, including the asbestos fibers.

The invention also relates to a soil improver in the form of a solid body comprising partly dried sludge and fungi applied therein, including one or more of the following fungi that break down undesirable asbestos fibers: Verticillium leptobactrum, Talaromyces flavus, Trametes versicolor, Aspergillus tubingenesis and Postia stiptica. With regard to the soil improver, the invention is characterized in that this soil improver furthermore contains seeds or roots of a plant that has a high biomass production and fungi naturally associated with this plant.

Preferably, the soil improver contains one or more fungi naturally associated with the following plants: Miscanthus, Hemp, Sorghum, and Nettles.

An embodiment of the soil improver according to the invention is characterized in that it further comprises one or more seeds or roots of the following plants: Miscanthus, Hemp, Sorghum and nettles.

Preferably, the soil improver according to the invention furthermore comprises one or more of the following further living organisms: worms, worm eggs and predatory mites. The product is therefore not only a carrier of nutrients as a soil improver, but also of biological control methods in the form of livestock. Due to the nature and shape of the product, these species can even be introduced in the form of spores, eggs, or larvae, 'germinating' as it were when they are applied in agriculture.

Furthermore, the sludge in the soil improver is preferably river sediment. River sediment is a very good carrier for the application of living matter because it comes from an anaerobic environment and makes a transition to an aerobic environment, whereby the 'old life' in the river sediment dies completely, while the chemical and organic composition is preserved. but at the same time a virgin/clean environment is created, which lends itself well to introducing the new life necessary to create a healthy soil.

### Brief description of the drawings

The invention will be further elucidated below with reference to test results shown in the drawing, wherein:

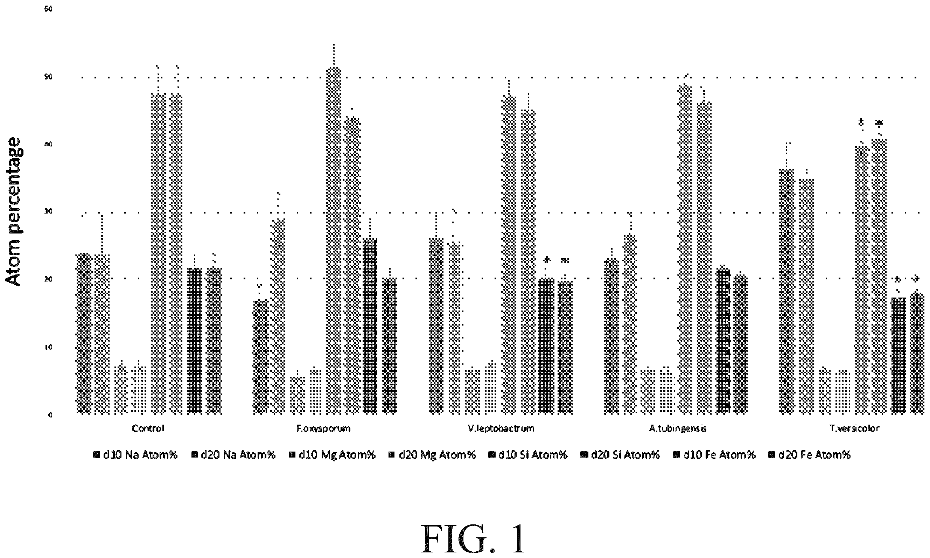

Figure 1 is a graph showing the results of incubations with crocidolite fibers and four different fungal species.

### Detailed description of the drawings

In several methods of breaking down asbestos fibers in the soil, Verticillium leptobactrum, Talaromyces flavus, Postia stiptica and a combination of one or more of these fungi have been introduced into the soil. In addition, Miscanthus, Hemp, Sorghum, nettles and a combination of one or more of these plants were planted in the soil. These plants are very suitable for mining the soil.

Taking into account the observation that potting soil disappears with the growth of the root mass, it can be assumed that Si, an important component of garden soil, can also be taken up by the plant roots. This is in agreement with the observation that a tree can grow, as it were, in a bare rock. These observations make it highly probable that there are thus conceivable conditions under which chrysotile as a whole can break down into its constituent chemical elements, as long as the right factors are available. A mycorrhiza of the right fungus with the right plant root in contact with asbestos fibers is one such factor.

The fungi cannot pass on the undesirable substances from the soil to the plant, with which they normally have a mutualistic relationship and, among other things, receive the oxygen and sugars that are also necessary to make the decomposition possible.

By linking the fungi to a plant, so that a combination of the 'pressing' plant and the effectively degrading fungi is formed, there is strong pressure on the available medium (soil contaminated with asbestos).

The plant, together with the "native" microbial flora, will completely exhaust the nutrients and minerals due to the extremely high root density. The combination of acids, siderophores and other secondary metabolites by the plant and microorganism along with the continuous uptake of metals and minerals by the plant causes the "disappearance" of organic and mineral components from the soil, including the asbestos fibers.

To demonstrate that the fungi break down asbestos, a test was carried out with four different types of fungus. Figure 1 shows the results of incubations with crocidolite fibers and the four different fungal species. Shown are the atomic percentages of Na, Mg, Si and Fe measured in 25 fibers per sample using SEM-Xray microelement analysis. Per bar the mean of 4 replicates is shown from incubations stopped to 10 and 20 days, respectively. * indicates significant differences from control samples without fungi, tested by one-way ANOVA and Ttest.

The results in figure 1 show that modifications of both the Si and the Fe content already occur after 10 days in two fungal species. In Trametes versicolor already after 10 days, while in Verticillium leptobactrum this is the case after 20 days. However, other modifications are also observable. Thus, in the case of Fusarium oxysporum, we see that in time, day 10 vs. 20, a decrease in Si and Fe can be seen in the incubations with the fungus. After analyzing some of the samples, it was concluded that the incubation with fungi leads to a reduction in the iron and silicon content of the fibers and thus to a modification of the fibers. The visual observation that fungi grew better in the presence of asbestos fibers indicates that the fungi extract essential building blocks for growth from the fibers. Based on these results, it can be established that asbestos fibers can be successfully broken down with the help of fungi.

An embodiment of the method according to the invention which is suitable for breaking down undesired asbestos fibers in the soil is described below. Fe and Mg (and to a lesser extent Si) act as necessary trace elements for these undesirable fibers, especially asbestos. By removing Fe and Mg from the unwanted fibers (asbestos), the fiber structure is broken down, so that the fiber is in fact no longer the harmful fiber it used to be. Fe and Mg are extracted from the unwanted fibers by the amino acids secreted by the fungi.

The product that is added to the soil to be cleaned is on the one hand a combination of the fungi that break down asbestos and the fungi that naturally belong to Miscanthus. But the latter are not aimed at the degradation of asbestos. So you need both and the asbestos-degrading fungi have to pass their decomposition material to the fungi of the Miscanthus who then supply them to the Miscanthus.

It is very important that in the process a scarcity of nutrients is created (i.e. the soil is emptied), so that the fungi and the plant - when all the freely absorbable Mg, Fe has disappeared - then go on the hunt for the difficult-to-absorb Fe and Mg as it is stuck in the fibers. Miscanthus is very attractive for this because it can last for years and if it is harvested year after year, the soil is impoverished and the work easier for the fungi.

## Claims

1. A method for breaking down undesired asbestos fibers in the soil by introducing one or more of the following fungi into the soil to be cleaned: Verticillium leptobactrum, Talaromyces flavus, Trametes versicolor, Aspergillus tubingenesis and Postia stictica, **characterized in that** fungi are also introduced into the soil which belong naturally to a plant that has a high biomass production and that this plant is planted in the soil, whereby no nutrients are added to the soil.

2. Method according to claim 1, **characterized in that** the plant with high biomass production is harvested periodically.

3. Method according to claim 1 or 2, **characterized in that** as a plant with high biomass production, the Miscanthus plant is planted in the soil and the fungi naturally associated with this plant are introduced into the soil.

4. Method according to claim 1, 2, or 3, **characterized in that** one or more of the following plants are furthermore arranged in the soil to be cleaned: Hemp, Sorghum, bamboo, hops and nettles.

5. A soil improver in the form of a solid body comprising partly dried sludge and fungi introduced therein, including one or more of the following fungi that break down undesirable asbestos fibers: Verticillium leptobactrum, Talaromyces flavus, Trametes versicolor, Aspergillus tubingenesis and Postia stiptica, **characterized in that** the soil improver further contains seeds or roots of a plant having a high biomass production and fungi naturally associated with this plant.

6. A soil improver according to claim 5, **characterized in that** the soil improver contains one or more fungi naturally associated with the following plants: Miscanthus, Hemp, Sorghum, and Nettles.

7. A soil improver according to claim 6, **characterized in that** the soil improver further comprises one or more seeds or roots of the following plants: Miscanthus, Hemp, Sorghum and nettles.

8. A soil improver according to claim 6 or 7, **characterized in that** the soil improver further comprises one or more of the following further living organisms: worms, worm eggs and predatory mites.

9. A soil improver according to claim 5, 6, 7 or 8, **characterized in that** the sludge is river sediment.

## Patentansprüche

1. Verfahren zum Abbau unerwünschter Asbestfasern im Boden durch Einbringen eines oder mehrerer der folgenden Pilze in den zu reinigenden Boden: Verticillium leptobactrum, Talaromyces flavus, Trametes versicolor, Aspergillus tubingenesis und Postia stictica, **dadurch gekennzeichnet, dass** auch Pilze in den Boden eingebracht werden, die von Natur aus zu einer Pflanze gehören, die eine hohe Biomasseproduktion aufweist, und dass diese Pflanze in den Boden gepflanzt wird, wobei dem Boden keine Nährstoffe zugeführt werden.

2. Verfahren nach Anspruch 1, **dadurch gekennzeichnet, dass** die Pflanze mit hoher Biomasseproduktion periodisch geerntet wird.

3. Verfahren nach Anspruch 1 oder 2, **dadurch gekennzeichnet, dass** die Miscanthus-Pflanze als Pflanze mit hoher Biomasseproduktion in den Boden gepflanzt wird und die mit dieser Pflanze natürlicherweise verbundenen Pilze in den Boden eingebracht werden.

4. Verfahren nach Anspruch 1, 2 oder 3, **dadurch gekennzeichnet, dass** im zu reinigenden Boden außerdem eine oder mehrere der folgenden Pflanzen angeordnet werden: Hanf, Sorghum, Bambus, Hopfen und Brennnesseln.

5. Bodenverbesserungsmittel in Form eines festen Körpers, bestehend aus teilweise getrocknetem Schlamm und darin eingebrachten Pilze, einschließlich eines oder mehrerer der folgenden Pilze, die unerwünschte Asbestfasern abbauen: Verticillium leptobactrum, Talaromyces flavus, Trametes versicolor, Aspergillus tubingenesis und Postia stiptica, **dadurch gekennzeichnet, dass** das Bodenverbesserungsmittel außerdem Samen oder Wurzeln einer Pflanze mit einer hohen Biomasseproduktion und mit dieser Pflanze natürlicherweise assoziierte Pilze enthält.

6. Bodenverbesserer nach Anspruch 5, **dadurch gekennzeichnet, dass** der Bodenverbesserer einen oder mehrere Pilze enthält, die natürlicherweise mit den folgenden Pflanzen assoziiert sind: Miscanthus, Hanf, Sorghum und Brennnessel.

7. Bodenverbesserer nach Anspruch 6, **dadurch gekennzeichnet, dass** der Bodenverbesserer außerdem einen oder mehrere Samen oder Wurzeln der folgenden Pflanzen enthält: Miscanthus, Hanf, Sorghum und Brennnesseln.

8. Bodenverbesserer nach Anspruch 6 oder 7, **dadurch gekennzeichnet, dass** der Bodenverbesserer außerdem einen oder mehrere der folgenden weiteren lebenden Organismen umfasst: Würmer, Wurmeier und Raubmilben.

9. Bodenverbesserungsmittel nach Anspruch 5, 6, 7 oder 8, **dadurch gekennzeichnet, dass** der Schlamm Flusssediment ist.

## Revendications

1. Procédé pour décomposer les fibres d'amiante indésirables dans le sol par introduction d'un ou plusieurs des champignons suivants dans le sol à nettoyer: Verticillium leptobactrum, Talaromyces flavus, Trametes versicolor, Aspergillus tubingenesis et Postia stictica, **caractérisé en ce que** les champignons sont également introduits dans le sol qui appartiennent naturellement à une plante ayant une production élevée de biomasse et que cette plante est plantée dans le sol, de sorte qu'aucun élément nutritif n'est ajouté au sol.

2. Procédé selon la revendication 1, **caractérisé en ce que** la plante à forte production de biomasse est récoltée périodiquement.

3. Procédé selon la revendication 1 ou 2, **caractérisé en ce qu'**en tant que plante à forte production de biomasse, la plante Miscanthus est plantée dans le sol et les champignons naturellement associés à cette plante sont introduits dans le sol.

4. Procédé selon la revendication 1, 2 ou 3, **caractérisé en ce qu'**on dispose en outre dans le sol à nettoyer une ou plusieurs des plantes suivantes: Chanvre, Sorgho, bambou, houblon et orties.

5. Amendement du sol sous la forme d'un corps solide comprenant des boues partiellement séchées et des champignons introduits à l'intérieur, y compris un ou plusieurs des champignons suivants qui décomposent les fibres d'amiante indésirables: Verticillium leptobactrum, Talaromyces flavus, Trametes versicolor, Aspergillus tubingenesis et Postia stiptica, **caractérisé en ce que** l'amendement du sol contient en outre des graines ou des racines d'une plante ayant une production élevée de biomasse et des champignons naturellement associés à cette plante.

6. Amendement de sol selon la revendication 5, **caractérisé en ce que** l'amendement de sol contient un ou plusieurs champignons naturellement associés aux plantes suivantes : Miscanthus, Chanvre, Sorgho et Orties.

7. Amendement de sol selon la revendication 6, **caractérisé en ce que** l'amendement de sol comprend en outre une ou plusieurs graines ou racines des plantes suivantes: Miscanthus, Chanvre, Sorgho et orties.

8. Amendement de sol selon la revendication 6 ou 7, **caractérisé en ce que** l'amendement de sol comprend en outre un ou plusieurs des autres organismes vivants suivants: vers, oeufs de vers et acariens prédateurs.

9. Amendement de sol selon la revendication 5, 6, 7 ou 8, **caractérisé en ce que** les boues sont des sédiments fluviaux.
